# EUROPEAN PATENT APPLICATION

(11) **EP 3 702 767 A1**
(43) Date of publication of application: **02.09.2020**
(21) Application number: 18869820.3
(22) Date of filing: 25.10.2018
(51) Int. Cl.: G01N 24/00, A61B 5/055, A61K 49/10, G01N 24/12

(54) **COMPOSITION, COMPOSITION FOR DYNAMIC NUCLEAR POLARIZATION, HIGH POLARIZATION METHOD, HIGHLY POLARIZED SUBSTANCE, AND NMR MEASURING METHOD**

(30) Priority: 26.10.2017 JP 2017207239
(71) Applicant: Kyushu University, National University Corporation, Fukuoka-shi, Fukuoka 819-0395 (JP); Riken, Wako-shi, Saitama 351-0198 (JP)
(72) Inventor: YANAI Nobuhiro, Fukuoka-shi Fukuoka 819-0395 (JP); KIMIZUKA Nobuo, Fukuoka-shi Fukuoka 819-0395 (JP); HOSOYAMADA Masanori, Fukuoka-shi Fukuoka 819-0395 (JP); FUJIWARA Saiya, Fukuoka-shi Fukuoka 819-0395 (JP); TATEISHI Kenichiro, Wako-shi Saitama 351-0198 (JP); UESAKA Tomohiro, Wako-shi Saitama 351-0198 (JP)
(74) Representative: Zimmermann & Partner Patentanwälte mbB
(86) International application number: PCT/JP2018/039591
(87) International publication number: WO 2019/082951

(57) **Abstract**

A composition containing (1) a porous material and (2) a polarization source for dynamic nuclear polarization containing a molecule capable of being in an excited triplet state. According to the composition, a dynamic nuclear polarization system that has a long spin-lattice relaxation time and can readily introduce the polarization object thereto can be provided.

## Description

### Technical Field

The present invention relates to a composition capable of enhancing spin polarization of nuclei, a polarization enhancing method using the composition, a substance that is highly polarized by the polarization enhancing method, and an NMR measurement method for the substance.

### Background Art

Atomic nuclei having a magnetic moment (i.e., nuclear spins) precess in a magnetostatic field, and under irradiation with an electromagnetic wave having the same frequency as the precession movement, a nuclear magnetic resonance (NMR) phenomenon emerges through absorption of the energy of the electromagnetic field with resonance of the nuclear spins. The resonance frequency of the NMR phenomenon varies depending on the nuclear species and the chemical or magnetic environment, to which the atomic nuclei are exposed, and accordingly, in the fields of organic chemistry and biochemistry, the NMR spectroscopy, in which the frequency spectrum of the NMR signals obtained by converting the energy absorption through the resonance to electric signals (i.e., the chemical shift values) is observed for analyzing the molecular structures and the properties of compounds, has been frequently performed. In the field of medical, the magnetic resonance imaging (MRI), in which the NMR signals are imaged with the positional information, has been applied to the non-invasive test for biological organs, such as a brain.

The assembly of the nuclear spins, to which a magnetostatic field is applied, is split, for example in the case of protons, into the energy state where the spins are directed in parallel to the magnetic field and the energy state where the spins are directed in anti-parallel to the magnetic field. Herein, the value obtained by dividing the difference between the numbers of spins having the energy states (i.e., the occupation numbers) by the total number of spins is referred to as a spin polarization, and it has been said that the intensity of the NMR signal is proportional to the polarization. However, the polarization of the nuclear spins at room temperature is generally an extremely small value of several ten thousandths, which is a factor restricting the sensitivity of the NMR spectroscopy and the MRI.

Under the circumstances, as a method for enhancing the polarization of the nuclear spins, there has been a proposal of a dynamic nuclear polarization method, in which the spin polarization of electrons is transferred to the surrounding nuclei through the solid effect induced through electromagnetic wave irradiation, so as to enhance the polarization of the nuclear spins. As the supply source of the electron spins, radicals and photoexcited triplet molecules are used, and among these, the photoexcited triplet molecules have an advantage capable of effectively enhancing the polarization of the nuclear spins at room temperature since the occupation number of the electron spins is largely polarized to the particular energy state irrespective of the temperature.

As for the dynamic nuclear polarization system using the photoexcited triplet molecules, NPL 1 describes *p*-terphenyl as an organic crystal substrate having added thereto pentacene as the photoexcited triplet molecule, and NPL 2 proposes *o*-terphenyl as an amorphous substrate having added thereto pentacene as the photoexcited triplet molecule.

### Citation List

### Non-patent Literatures

NPL 1: PNAS, 2014, 111, 7529
NPL 2: Angew. Chem. Int. Ed., 2013, 52, 13307

### Summary of Invention

### Technical Problem

As described above, NPL 1 proposes the dynamic nuclear polarization system including an organic crystal substrate of *p*-terphenyl having added thereto pentacene as the photoexcited triplet molecule, and NPL 2 proposes the dynamic nuclear polarization system including an amorphous substrate of *o*-terphenyl having added thereto pentacene as the photoexcited triplet molecule. However, these dynamic nuclear polarization systems may not be sufficiently satisfactory and may require further improvements for practical realization.

Specifically, the dynamic nuclear polarization system using an organic crystal substrate has a long spin-lattice relaxation time of 300 seconds or more due to the rigidity of the crystal skeleton, and it is considered that the spin polarization transferred from the electrons to the nuclei is readily accumulated to enhance the polarization of the nuclear spins. However, the rigidity of the crystal skeleton may provide a problem of difficulty in introduction of the polarization source and the polarization object into the substrate. In the dynamic nuclear polarization system using an amorphous substrate, on the other hand, the polarization source and the polarization object can be relatively readily introduced, but o-terphenyl has a glass transition temperature of -30°C, which requires a cooling equipment. Polystyrene or the like having a glass transition temperature of 20°C or more (room temperature) has a short spin-lattice relaxation time of 10 seconds or less, and thus has a problem that the nuclear spins cannot be sufficiently polarized due to the difficulty in accumulation of the nuclear spin polarization.

For solving these technical problems, the present inventors have made investigations for providing a dynamic nuclear polarization system that has a long spin-lattice relaxation time and can readily introduce the polarization object thereto.

### Solution to Problem

As a result of the earnest investigations for solving the problems, the inventors have found that a dynamic nuclear polarization system that has a long spin-lattice relaxation time and can readily introduce the polarization source and the polarization object thereto can be achieved by combining (1) a porous material and (2) a polarization source formed of a molecule capable of being in an excited triplet state. The invention is proposed based on the knowledge, and specifically includes the following embodiments.
[1] A composition containing (1) a porous material and (2) a polarization source for dynamic nuclear polarization containing a molecule capable of being in an excited triplet state.
[2] The composition according to the item [1], wherein the molecule capable of being in an excited triplet state is a compound having a skeleton containing from 4 to 6 benzene rings condensed to each other.
[3] The composition according to the item [2], wherein the molecule capable of being in an excited triplet state is a compound having a pentacene skeleton.
[4] The composition according to any one of the items [1] to [3], wherein the molecule capable of being in an excited triplet state is a compound constituted only by a carbon atom, a hydrogen atom, and a deuterium atom.
[5] The composition according to any one of the items [1] to [4], wherein the porous material is a metal-organic framework or a covalent organic framework.
[6] The composition according to the item [5], wherein the composition contains the metal-organic framework, the metal-organic framework has an organic ligand that has a ring structure substituted by a substituent, and at least one hydrogen atom of the substituent is substituted by deuterium.
[7] The composition according to the item [5], wherein the composition contains the metal-organic framework, and the metal-organic framework has an organic ligand that has an imidazole skeleton.
[8] The composition according to any one of the items [5] to [7], wherein the composition contains the metal-organic framework, and the metal-organic framework has a metal ion that contains a divalent to tetravalent metal ion.
[9] The composition according to any one of the items [5] to [8], wherein the composition contains the metal-organic framework, and the metal-organic framework has a metal ion that contains a zinc ion Zn²⁺.
[10] The composition according to any one of the items [5] to [9], wherein the composition contains the metal-organic framework, and the molecule capable of being in an excited triplet state has a functional group that interacts with a metal ion of the metal-organic framework.
[11] The composition according to the item [10], wherein the functional group is an acidic group.
[12] The composition according to the item [10], wherein the functional group is a carboxy group or a carboxylate anion group.
[13] The composition according to any one of the items [1] to [12], wherein the molecule capable of being in an excited triplet state exists in pores of the porous material.
[14] The composition according to any one of the items [5] to [13], wherein the composition has a content of the polarization source of 0,01% by mol or more with respect to the molar number of a metal ion of the metal-organic framework.
[15] The composition according to any one of the items [1] to [14], wherein the composition has a spin-lattice relaxation time *T*₁ of 10 seconds or more.
[16] The composition according to any one of the items (1) to [15], wherein the composition further contains a substance capable of receiving nuclear spin polarization formed in the polarization source and the porous material.
[17] A highly polarized composition containing the composition according to any one of the items [1] to [16].
[18] A highly polarized composition containing the composition according to any one of the items [1] to [16] that is highly polarized.
[19] A polarization enhancing method of a substance, including: contacting the substance with the highly polarized composition according to the item [18], or including: contacting the substance with the composition according to any one of the items [1] to [16]; and then highly polarizing the composition to form a highly polarized composition.
[20] The polarization enhancing method according to the item [19], wherein the substance is a liquid or a solution.
[21] The polarization enhancing method according to the item [19], wherein the substance is a gas.
[22] The polarization enhancing method according to the item [20] or [21], wherein the substance is contacted with the highly polarized composition or the composition by impregnating the porous material constituting the highly polarized composition or the composition, with the substance.
[23] The polarization enhancing method according to any one of the items [19] to [22], wherein the substance contains at least one kind of a compound selected from a hydrocarbon and a derivative of a hydrocarbon having at least one hydrogen atom substituted by a substituent.
[24] The polarization enhancing method according to the item [23], wherein the substance contains the derivative of a hydrocarbon having at least one hydrogen atom substituted by a substituent, and at least one of the substituent contains an atom having a spin quantum number I other than 0.
[25] The polarization enhancing method according to the item [23] or [24], wherein the substituent is a fluorine atom.
[26] The polarization enhancing method according to any one of the items [19] to [25], wherein the method further includes transferring nuclear spin polarization of the highly polarized composition to the substance.
[27] The polarization enhancing method according to the item [26], wherein the nuclear spin polarization of the highly polarized composition is transferred to the substance by irradiating the highly polarized composition and the substance contacted with each other, with a microwave.
[28] A highly polarized substance highly polarized by the method according to any one of the items [18] to [27].
[29] An NMR measurement method including: measuring NMR of a substance with the composition according to any one of the items [1] to [16].

### Advantageous Effects of Invention

According to the composition of the invention, a dynamic nuclear polarization system that has a long spin-lattice relaxation time and can readily introduce the polarization source and the polarization object thereto can be achieved. The use of the composition can enhance the polarization of nuclear spins through the enhancement of the polarization of various substances, and can effectively enhance the sensitivity of NMR measurement thereof.

### Brief Description of Drawings

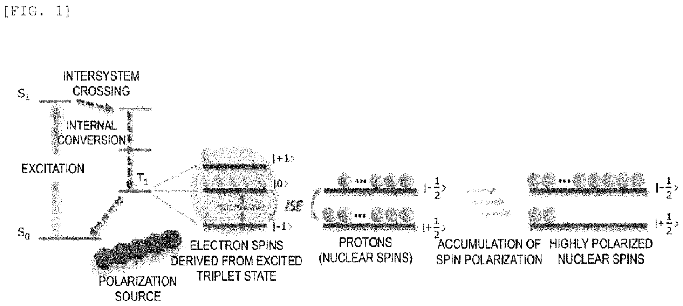

Fig. 1 is a schematic illustration showing the dynamic nuclear polarization mechanism of the composition of the invention.

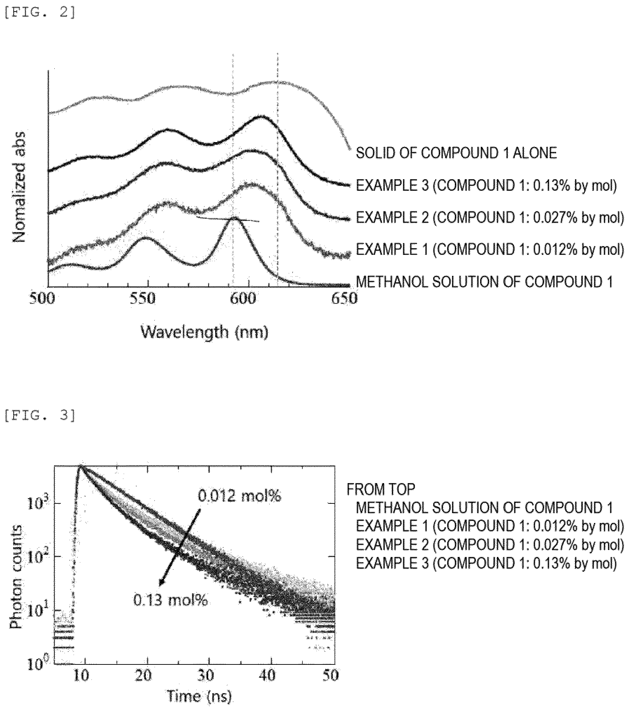

Fig. 2 is the light absorption spectra of the composition of Example 1 containing MOF having introduced thereto 0.012% by mol of the compound 1, the composition of Example 2 containing MOF having introduced thereto 0.027% by mol of the compound 1, the composition of Example 3 containing MOF having introduced thereto 0.13% by mol of the compound 1, a solid of the compound 1 alone, and a methanol solution of the compound 1.
Fig. 3 is the transient decay curves of fluorescent light emission of the composition of Example 1 containing MOF having introduced thereto 0.012% by mol of the compound 1, the composition of Example 2 containing MOF having introduced thereto 0,027% by mol of the compound 1, the composition of Example 3 containing MOF having introduced thereto 0.13% by mol of the compound 1, a solid of the compound 1 alone, and the compound 1.

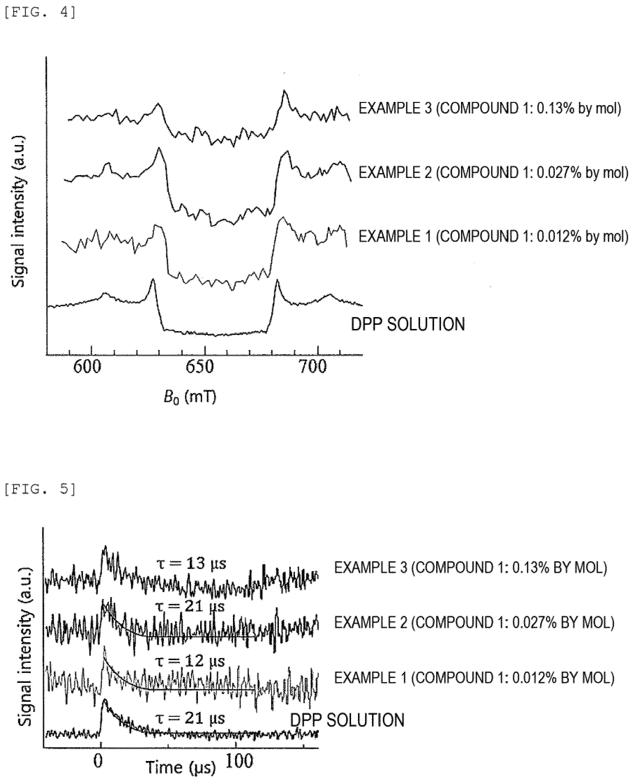

Fig. 4 is the electron spin resonance spectra of the composition of Example 1 containing MOF having introduced thereto 0.012% by mol of the compound 1, the composition of Example 2 containing MOF having introduced thereto 0.027% by mol of the compound 1, the composition of Example 3 containing MOF having introduced thereto 0.13% by mol of the compound 1, and a chloroform solution of DPP.
Fig. 5 is the transient decay curves of the ESR peak at 680 mT of the composition of Example 1 containing MOF having introduced thereto 0.012% by mol of the compound 1, the composition of Example 2 containing MOF having introduced thereto 0.027% by mol of the compound 1, the composition of Example 3 containing MOF having introduced thereto 0.13% by mol of the compound 1, and a chloroform solution of DPP.

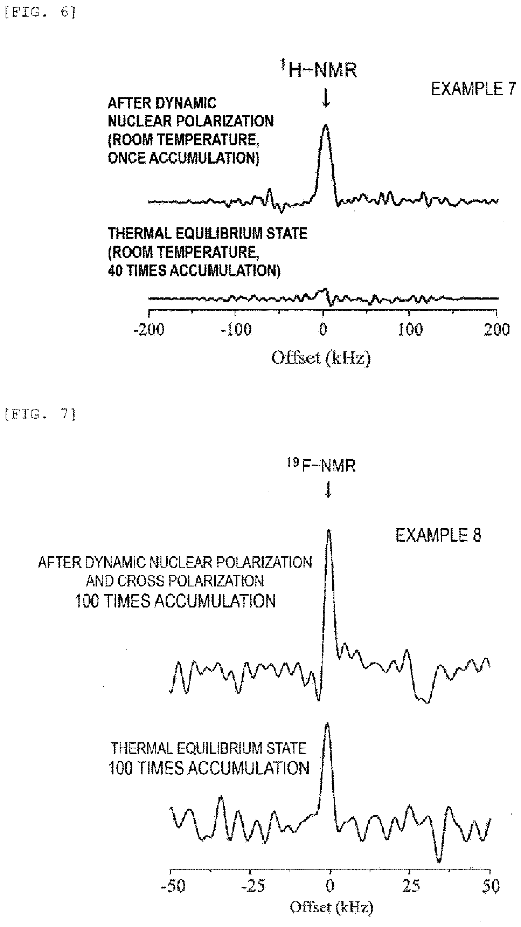

Fig. 6 is the NMR spectra showing the ¹H-NMR signals observed from the composition of Example 7 containing MOF using the ligand 2 having introduced thereto the compound 1.
Fig. 7 is the NMR spectra showing the ¹⁹F-NMR signals observed from the compound of Example 8 containing the composition of Example 7 having introduced thereto the object 1.

### Description of Embodiments

The contents of the invention will be described in detail below. The constitutional elements may be described below based on representative embodiments and specific examples, but the invention is not limited to the embodiments or the specific examples. In the description herein, the numeral range shown with "to" means the range that includes the numerals shown before and after "to" as the lower limit value and the upper limit value. The isotope species of the hydrogen atom existing in the molecule of the compound used in the invention is not particularly limited, and for example, all the hydrogen atoms in the molecule may be ¹H, and a part or the whole thereof may be ²H (i.e., deuterium D). The "excitation light" means light that causes light emission of the object through excitation of the object, and light having a wavelength that agrees with the absorption wavelength of the object may be used therefor.

### <Composition>

The composition of the invention contains (1) a porous material and (2) a polarization source for dynamic nuclear polarization containing a molecule capable of being in an excited triplet state.

The "porous material" in the invention means a material that is in a solid state at 20°C and 1 atm, and has plural pores in the solid state. The pores of the porous material may be either through pores or non-through pores.

In dynamic nuclear polarization, in which the polarization of nuclear spins is enhanced by transferring spin polarization of electrons thereto through irradiation of an assembly of the electron spins in a polarized state and the nuclear spins with an electromagnetic wave under application of an external magnetic field, the "polarization source" in the invention means the supply source of the electron spins in a polarized state. The "polarization" or "spin polarization" herein means that in the Zeeman splitting of an assembly of spins under application of a magnetostatic field, the occupation numbers of spins in the energy levels are different from each other between the split energy levels. Taking a combination of two energy levels from the split energy levels, the ratio of the difference between the occupation number N₁ of spins in one of the energy levels and the occupation number N₂ of spins in the other of the energy levels with respect to the total number of spins, i.e., (N₁-N₂)/(N₁+N₂), is referred to as a polarization. The case where the polarization is 0 in all the combinations of the energy levels undergoing Zeeman splitting can be said that no spin polarization occurs, and the case where the polarization exceeds 0 in at least one combination can be said that spin polarization occurs. A larger polarization means larger spin polarization as spins exist excessively in any one of the energy levels. In the description below, the energy levels undergoing Zeeman splitting each are referred to as a "Zeeman level". The number of Zeeman levels of electrons and nuclei in the composition of the invention may be two or three or more. In the case where the number of Zeeman levels is three or more, the polarization that exceeds 0 in the state of spin polarization may be in all the combinations of the Zeeman levels or in a part of the combinations thereof.

In the invention, the "molecule capable of being in an excited triplet state" is used as the "polarization source". The "molecule capable of being in an excited triplet state" means a molecule that is capable of undergoing transition to an excited triplet state under application of excitation energy. The transition to an excited triplet state may be transition from the ground singlet state to an excited triplet state occurring directly through the application of excitation energy, or may be intersystem crossing from an excited singlet state occurring through the application of excitation energy to an excited triplet state. The excitation energy causing the transition to an excited state may be either energy of excitation light, recombination energy of injected carriers, or excitation energy received from other molecules in an excited state. The polarization state of electron spins of the molecule capable of being in an excited triplet state will be described in the section [1] Excitation Process of Polarization Source below.

The composition of the invention contains the porous material and the polarization source containing the molecule capable of being in an excited triplet state described above, and may encompass the polarization source and the polarization object in the pores of the porous material. According to the embodiment, the polarization source and the polarization object can be readily introduced to the interior of the porous material, and can be retained in a spatially dispersed state. The porous material can encompass the polarization source and the polarization object in the pores through introduction thereto, and therefore, in consideration of the introduction thereof, a flexible material, such as an amorphous material, may not be necessarily selected, but a material may be selected by its spin-lattice relaxation time. Accordingly, the composition of the invention can achieve dynamic nuclear polarization system that has a long spin-lattice relaxation time and can readily introduce the polarization object.

The composition of the invention can achieve the polarization of electron spins having the occupation numbers of the electron spins that are largely polarized to the particular energy level, irrespective of the temperature, due to the use of the molecule capable of being in an excited triplet state as the polarization source. Accordingly, even in the case where the polarization is enhanced at room temperature, the polarization of nuclear spins can be efficiently enhanced, and thus the operations and equipments relating to the temperature control can be simplified.

The dynamic nuclear polarization mechanism in the composition of the invention will be described with reference to Fig. 1 for the case where a polarization source is excited through irradiation of excitation light as an example. However, the dynamic nuclear polarization mechanism of the composition of the invention should not be construed as being limited to the mechanism described below.

### [1] Excitation Process of Polarization Source

In this process, the composition is irradiated with excitation light to cause transition of the polarization source formed of the molecule capable of being in an excited triplet state, to an excited triplet state.

In the irradiation of the composition with excitation light, as shown in Fig. 1, the polarization source transits from the ground singlet state So to the excited singlet state S₁, and further transits to the excited triplet state Tₙ through intersystem crossing from the excited singlet state S₁. Subsequently, the excited triplet state Tₙ is internally converted in a stepwise manner to lower excited triplet states, and finally becomes the excited triplet state *T*₁ of the lowest energy level. The number of Zeeman levels of the electron spins in the excited triplet state *T*₁ (i.e., the triplet electron spins) is three corresponding to the magnetic quantum numbers m = -1, 0, and +1, and the highly polarized state of electron spins is achieved, in which the electron spins distribution is largely polarized to the Zeeman level of m = 0 among these. On the other hand, the number of Zeeman levels of the nuclei of the porous material is, for example, two corresponding to the magnetic quantum numbers m = +1/2 and -1/2 in proton ¹H. Among these, the Zeeman level of m = +1/2 has a slightly larger occupation number of nuclear spins, but the polarization is approximately 10⁻⁶, which means an extremely low polarization state of the nuclear spins.

### [2] Polarization Enhancing Process

In this process, the polarization of the triplet electron spins formed in the composition is transferred to the nuclear spins to enhance the polarization of the nuclear spins.

In the irradiation of the composition having the triplet electron spins formed therein with an electromagnetic wave causing resonance with the electron spins under application of an external magnetic field, the solid effect changes the angle of the precession movement through resonance of the electron spins, and simultaneously the rate of change of the precession movement of the electron spins resonate with the surrounding nuclear spins. According thereto, corresponding to the frequency of the electromagnetic wave, for example, the electron spins in the Zeeman level of m = 0 drop to the Zeeman level of m = -1, simultaneously the nuclear spins in the Zeeman level of m = +1/2 in the porous material rise to the Zeeman level of m = -1/2, and during the irradiation with the electromagnetic wave, the cycle is repeated to exchange the spin polarization of the electrons and the spin polarization of the nuclei, which results in the enhancement of the polarization of the nuclear spins. At this time, in the invention, the highly polarized nuclear spins are accumulated and diffused due to the long spin-lattice relaxation time of the porous material, which results in the enhancement of the polarization of the entire porous material. Accordingly, in the case where a polarization object is introduced to the porous material, the spin polarization is transferred also to the nuclei thereof, and consequently the polarization of the nuclear spins of the polarization object is highly enhanced.

The conditions for the polarization enhancement are not particularly limited, and for example, the intensity of the external magnetic field may be appropriately selected from a range of from 0.1 to 1 T, the frequency of the electromagnetic wave may be appropriately selected from a range of from 2 to 20 GHz, and the intensity of the electromagnetic field may be appropriately selected from a range of from 0.1 to 100 W.

The aforementioned processes [1] and [2] may be performed in such a manner that the process [1] is performed, and then the process [2] is performed, or in such a manner that the processes [1] and [2] are performed simultaneously. In the later case, the composition is irradiated simultaneously with the excitation light and the electromagnetic wave causing resonance with the electron spins, under application of an external magnetic field.

As described above, the composition of the invention can enhance the polarization of the nuclear spins thereof and the nuclear spins of the polarization object introduced to the composition, through the dynamic nuclear polarization. Accordingly, the composition of the invention can be effectively used as a composition for dynamic nuclear polarization.

The components contained in the composition of the invention and the properties of the composition will be described below.

### <Polarization Source>

The polarization source used in the invention is constituted by the molecule capable of being in an excited triplet state, and functions as a supply source of electron spins in the enhancement of polarization.

The molecule capable of being in an excited triplet state may be an inorganic molecule or an organic molecule, and is preferably an organic molecule.

Examples of the preferred organic molecule capable of being used as the polarization source include a compound having a skeleton containing from 4 to 6 benzene rings condensed to each other. Specific examples of the skeleton containing from 4 to 6 benzene rings condensed to each other include a tetracene skeleton, a pentacene skeleton, a hexacene skeleton, a rubrene skeleton, and a picene skeleton, and also include a skeleton having a structure containing two or more kinds of the skeletons connected to each other, and a skeleton having a structure containing the skeleton having a benzene ring, a naphthalene ring, or a biphenyl ring, connected thereto. The compound having a skeleton containing from 4 to 6 benzene rings condensed to each other may be an unsubstituted compound formed only of the skeleton, or may be a derivative having a structure containing the skeleton substituted by a substituent, and is preferably the derivative. For the preferred range and specific examples of the substituent capable of being substituted on the skeleton, reference may be made to the preferred range and specific examples of the substituent capable of being represented by R shown later. Among these, the substituent of the skeleton is preferably a group that interacts with at least any one of the atoms and the atomic groups constituting the porous material, and with at least any one of the ions in the case where the porous material contains the ions. In this manner, the molecules of the polarization source can be prevented from being aggregated to form an aggregate, and the molecules can be readily introduced to the pores of the porous material. For example, in the case where the porous material is a metal-organic framework, the compound as the polarization source preferably has a functional group that interacts with the metal ion of the metal-organic framework. Examples of the functional group include an acidic group, such as a carboxy group (-COOH), a sulfo group (-SO_{3H}), a phosphono group (-P(O)(OH)₂), and a phosphonooxy group (-OP(O)(OH)₂), and an anion group obtained by removing proton from the group through ionization, and the functional group is preferably a carboxy group or a carboxylate anion group.

In the compound used as the polarization source, it is preferred that at least a part of hydrogen atoms thereof are substituted by deuterium, and it is more preferred that from 30 to 70% of hydrogen atoms existing in the compound are substituted by deuterium. In this manner, the spin-lattice relaxation time of the polarization source can be prolonged to enhance effectively the polarization of the nuclear spins. Herein, the position in the compound that is substituted by deuterium is preferably a relatively movable position. For example, in the case where the skeleton having from 4 to 6 benzene rings condensed to each other constituting the compound has an atomic group (substituent) bonded via a single bond, it is preferred that at least a part of hydrogen atoms of the substituent are substituted by deuterium, and it is more preferred that all the hydrogen atoms thereof are substituted by deuterium. Examples of the substituent that is preferably substituted by deuterium include an alkyl group having a number of carbon atoms of from 1 to 20.

The compound used as the polarization source is preferably a pentacene derivative represented by the following general formula (A) or a salt thereof.

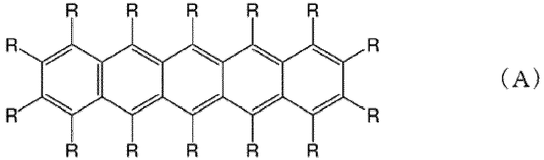

In the formula (A), R each independently represent a hydrogen atom (H), a deuterium atom (D), or a hydrocarbon group having a number of carbon atoms of from 1 to 20, which may contain at least one kind of an atom selected from the group consisting of an oxygen atom, a sulfur atom, and a silicon atom, provided that at least one of R represents a hydrocarbon group having a number of carbon atoms of from 1 to 20, which may contain at least one kind of an atom selected from the group consisting of an oxygen atom, a sulfur atom, and a silicon atom.

R in the formula (A) each independently represent a hydrogen atom (H), a deuterium atom (D), or a hydrocarbon group having a number of carbon atoms of from 1 to 20, which may contain at least one kind of an atom selected from the group consisting of an oxygen atom, a sulfur atom, and a silicon atom, and the "hydrocarbon group" is not limited to a linear saturated hydrocarbon group, but means that a carbon-carbon unsaturated bond, a branched structure, and a cyclic structure each may be contained therein. The expression "may contain at least one kind of an atom selected from the group consisting of an oxygen atom, a sulfur atom, and a silicon atom" means that a functional group containing an oxygen atom, a sulfur atom, or a silicon atom may be contained therein, and also means that a linking group containing an oxygen atom, a sulfur atom, or a silicon atom may be contained inside the carbon skeleton or at the end thereof. Therefore, the hydrocarbon group, which "may contain at least one kind of an atom selected from the group consisting of an oxygen atom, a sulfur atom, and a silicon atom" includes a hydrocarbon group having a number of carbon atoms of 2 containing a hydroxy group, such as -CH₂-CH₂-OH, a hydrocarbon group having a number of carbon atoms of 2 containing an ether group inside the carbon skeleton, such as -CH₂-O-CH₃, and a hydrocarbon group having a number of carbon atoms of 2 containing an ether group at the end of the carbon skeleton, such as -O-CH₂-CH₃.

The "salt" of the pentacene derivative means that the pentacene derivative is a compound having an acid center, such as a carboxy group (-COOH) and a sulfo group (-SO₃H), and the hydrogen ion thereof is replaced by a metal cation.

The expression "at least one of R represents a hydrocarbon group having a number of carbon atoms of from 1 to 20, which may contain at least one kind of an atom selected from the group consisting of an oxygen atom, a sulfur atom, and a silicon atom" means that the pentacene derivative represented by the formula (A) does not include pentacene itself and deuterated pentacene.

In the case where R represents a hydrocarbon group, the number of carbon atoms of the hydrocarbon group is preferably 3 or more, and more preferably 6 or more, and is preferably 12 or less, and more preferably 8 or less.

Examples of the hydrocarbon group include a phenyl group, a biphenyl group, a phenylthio group, a decylthio group, and an ethynyl group.

Examples of the functional group and the linking group contained in the hydrocarbon group include a carboxy group (-COOH), a potassium salt of a carboxy group (-COOK), a thioether group (-S-), a triethylsilyl group (-SiEt₃), and a triisopropylsilyl group (-SiiPr₃).

The number of the hydrocarbon group bonded is generally 1 or more, and preferably 2 or more, and is generally 6 or less, and preferably 5 or less.

The bonding positions of the hydrocarbon groups are preferably the combination of the 6-position and the 13-position (with a number of the hydrocarbon groups of 2), the combination of the 5-position, the 7-position, the 12-position, and the 14-position (with a number of the hydrocarbon groups of 4), the combination of the 1-position, the 4-position, the 8-position, and the 11-position (with a number of the hydrocarbon groups of 4), and the combination of the 2-position, the 3-position, the 9-position, and the 10-position (with a number of the hydrocarbon groups of 4), in which the combination of the 6-position and the 13-position is most preferred, and the combination of the 5-position, the 7-position, the 12-position, and the 14-position, the combination of the 1-position, the 4-position, the 8-position, and the 11-position, and the combination of the 2-position, the 3-position, the 9-position, and the 10-position are preferred in the descending order. The pentacene derivative has a tendency of undergoing oxidation decomposition when dissolving in a solvent in the air, and the decomposition rate is decreased in the above order depending on the positions where the hydrocarbon group is added. This corresponds to the descending order of the spin density of the π-electron cloud of pentacene.

It has been shown that the deuterium substitution of the hydrogen atom contained in pentacene enhances the ¹H spin polarization that is achieved by the dynamic nuclear polarization, as described in Proc. Natl. Acad. Sci, U.S.A., 2014, 111, 7527-7530.

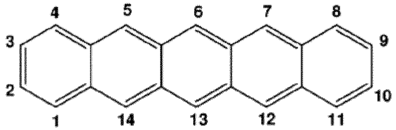

Examples of the pentacene derivative represented by the formula (A) and/or the salt thereof include the following compounds 1 to 11.

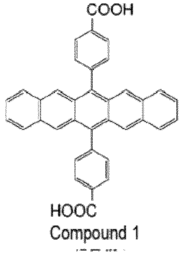

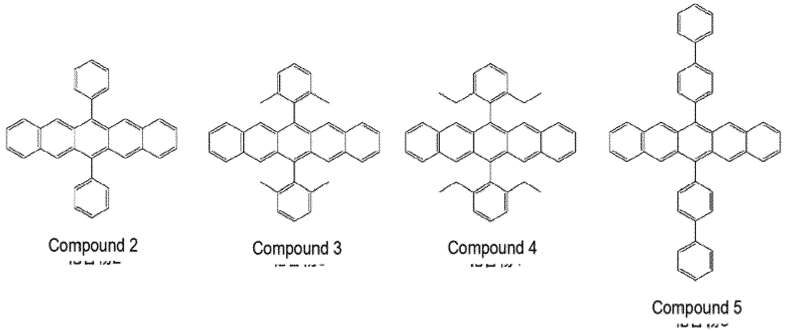

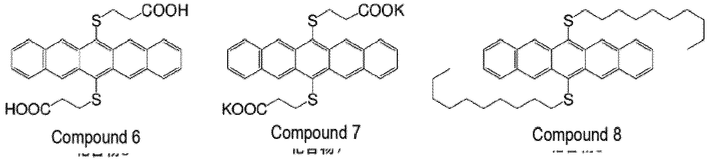

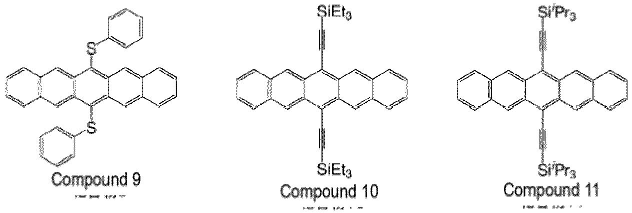

Among the compounds shown above, the compound 1 was measured for the electron spin resonance spectrum in the example shown later, and confirmed that an NMR signal was obtained therefrom.

Furthermore, the effect of the substituent influencing the HOMO-LUMO gap and the photooxidation resistance of pentacene compounds, for example, for the compounds 2, 3, 8, 9, and 11 shown above are described in detail in J. Am. Chem, Soc., 2008, 130, 16274-16286.

In addition, the usability of a pentacene derivative, such as the compounds 6 and 7, as the polarization source of dynamic nuclear polarization is shown in J. Mater. Chem. C, 2013, 1, 2193-2201.

In the eleven compounds shown above, a part or the whole of the hydrogen atoms (H) contained in the chemical formula may be substituted by deuterium (D).

A compound constituted only by a carbon atom, a hydrogen atom, and a deuterium atom may also be preferably used as the polarization source.

### [Porous Material]

The porous material used in the invention functions as a substrate that encompasses the polarization source in the pores thereof and retains the polarization source in a spatially dispersed state. The polarization source that is dispersed and retained can suppress the relaxation of the polarization of the triplet electron spins due to the aggregation of the molecules of the polarization source. The porous material in the invention further exerts the function receiving the spin polarization of the electrons of the polarization source in an excited triplet state, and accumulating and diffusing the spin polarization. In this manner, in the case where the polarization object is introduced into the porous material, the spin polarization in the porous material is transferred to the polarization object in the process of diffusing the spin polarization therein, and thus the polarization of the nuclear spins thereof is enhanced.

Examples of the porous material include a metal-organic framework (MOF), a crystalline porous polymer, such as a covalent organic framework (COF), an inorganic porous material, and an organic porous material, in which a metal-organic framework and a covalent organic framework are preferred, and a metal-organic framework is more preferred. In the metal-organic framework and the covalent organic framework, it is preferred that at least one hydrogen atom existing in the organic framework is substituted by deuterium, and it is more preferred that from 30 to 70% of hydrogen atoms existing in the organic framework are substituted by deuterium. In this manner, the spin-lattice relaxation time of the composition can be prolonged.

The metal-organic framework herein is a crystalline polymer framework having pores therein that is formed through continuous coordination bonds of a metal ion and a crosslinkable organic ligand. The metal-organic framework has a long spin-lattice relaxation time due to the hard crystalline skeleton thereof, and can effectively accumulate the spin polarization of nuclei to enhance the polarization. Furthermore, the metal-organic framework has pores having a nanosized small diameter, and thus can suppress the aggregation of the molecules of the polarization source and the polarization object by encompassing every individual molecule or every small number of molecules thereof in the pores. Moreover, the metal-organic framework has both the properties of a metal and an organic ligand, and thus can variously change in properties. According thereto, for example, the interaction between the metal-organic framework and the polarization source or the polarization object can be controlled to optimize the introduction amount and the state of the polarization enhancement thereof.

The metal ion constituting the metal-organic framework is not particularly limited, and is preferably a metal ion of a transition metal or a typical metal of Group 2, 13, or 14, more preferably an ion of copper, zinc, cadmium, silver, cobalt, nickel, iron, ruthenium, aluminum, chromium, molybdenum, manganese, palladium, rhodium, zirconium, titanium, magnesium, or lanthanum, further preferably an ion of zinc, aluminum, zirconium, or lanthanum, and particularly preferably a zinc ion or a zirconium ion. The metal ion may be used alone or as a combination of two or more kinds thereof.

The valence number of the metal ion is not particularly limited, and is preferably from divalent to hexavalent, more preferably from divalent to pentavalent, and further preferably from divalent to tetravalent.

The crosslinkable organic ligand is a coordinating compound having at least two coordinating groups. The plural coordinating groups of the organic ligand may be the same as or different from each other. The number of the coordinating groups of the organic ligand is preferably from 2 to 8, more preferably from 2 to 6, and further preferably from 2 to 4. Specific examples of the coordinating group include a coordinating nitrogen atom contained as a ring member of a nitrogen atom-containing heterocyclic ring, and a carboxy group. Among these, the carboxy group functions as a coordinating group in the form of a carboxylate anion group formed by removing proton through ionization.

The heterocyclic ring containing the coordinating nitrogen atom may be an aliphatic heterocyclic ring or an aromatic heterocyclic ring. Examples of the heterocyclic ring containing the coordinating nitrogen atom include an imidazole ring, a triazine ring, a pyridine ring, and a pyrimidine ring, in which an imidazole ring is preferred. The heterocyclic ring may be substituted by a substituent. For the preferred range and specific examples of the substituent, reference may be made to the preferred range and specific examples of the substituent represented by R¹ to R¹ shown later.

The organic framework substituted by the carboxy group as the coordinating group is not particularly limited, and examples thereof include an aromatic hydrocarbon ring, an alkene, a heterocyclic ring containing the coordinating nitrogen atom, an alkane, an alkyne, and a non-aromatic hydrocarbon ring. The organic framework may be substituted by a substituent. For the preferred range and specific examples of the substituent, reference may be made to the preferred range and specific examples of the substituent represented by R¹ to R³ shown later.

In the case where the organic ligand of the metal-organic framework has a ring structure substituted by a substituent, it is preferred that at least one hydrogen atom contained in the substituent is substituted by deuterium, and it is more preferred that all the hydrogen atoms thereof are substituted by deuterium.

Preferred examples of the crosslinkable organic ligand include an imidazole ligand represented by the following general formula (B).

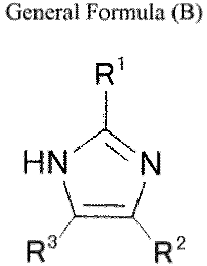

In the general formula (B), R¹ to R³ each independently represent a hydrogen atom or a substituent. In the case where two or more of R¹ to R³ each represent a substituent, the substituents may be the same as or different from each other. In the substituent in R¹ to R³, it is preferred that at least a part of hydrogen atoms contained in the substituent are substituted by deuterium, and it is more preferred that all the hydrogen atoms thereof are substituted by deuterium. According thereto, the spin-lattice relaxation time of the composition can be prolonged. The number of the substituent in R¹ to R³ is not particularly limited, and at least R¹ is preferably a substituent. The substituent represented by R¹ is preferably a substituted or unsubstituted alkyl group, more preferably an alkyl group, in which at least a part of hydrogen atoms are substituted by deuterium, and further preferably an alkyl group, in which all the hydrogen atoms are substituted by deuterium. The number of carbon atoms of the alkyl group is preferably from 1 to 20, more preferably from 1 to 4, and further preferably 1.

Examples of the substituent represented by R¹ to R³ include a hydroxy group, a halogen atom, an alkyl group having a number of carbon atoms of from 1 to 20, an alkoxy group having a number of carbon atoms of from 1 to 20, an alkylthio group having a number of carbon atoms of from 1 to 20, an alkyl-substituted amino group having a number of carbon atoms of from 1 to 20, an aryl-substituted amino group having a number of carbon atoms of from 1 to 20, an aryl group having a number of carbon atoms of from 6 to 40, a heteroaryl group having a number of carbon atoms of from 3 to 40, an alkenyl group having a number of carbon atoms of from 2 to 10, an alkynyl group having a number of carbon atoms of from 2 to 10, an alkylamide group having a number of carbon atoms of from 2 to 20, an arylamide group having a number of carbon atoms of from 7 to 21, an trialkylsilyl group having a number of carbon atoms of from 3 to 20, and an oligoethylene glycol group having a number of carbon atoms of from 2 to 20. In the specific examples, the group that can be further substituted by a substituent may be substituted.

Specific examples of the crosslinkable organic ligand include ligands represented by the following formulae. Specific examples thereof also include the ligands shown below that have a ring structure substituted by a methyl group, in which the hydrogen atoms of the methyl group are substituted by deuterium. However, the organic ligand of the metal-organic framework capable of being used as the porous material of the invention is not construed as being limited to the specific examples.

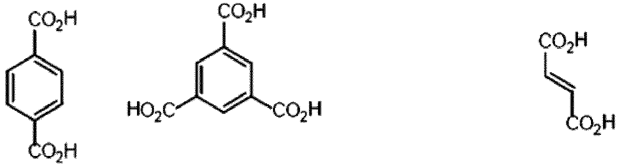

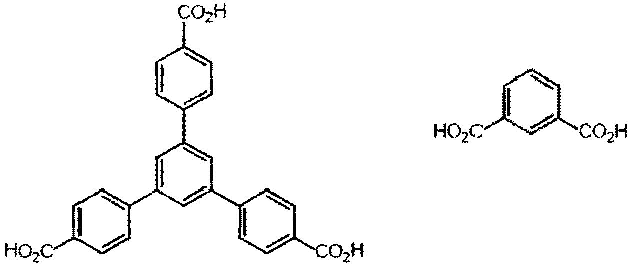

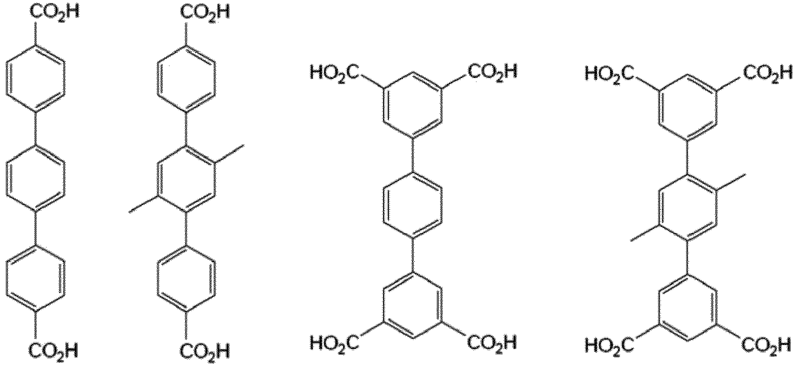

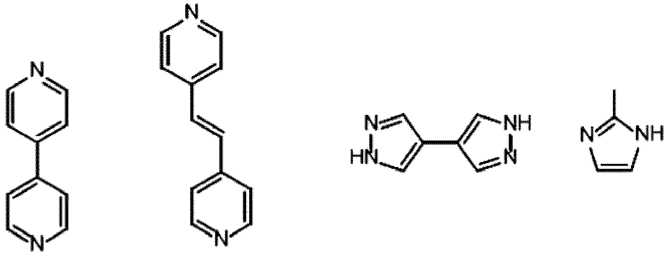

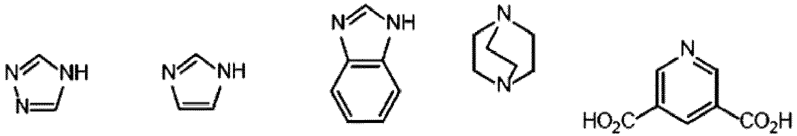

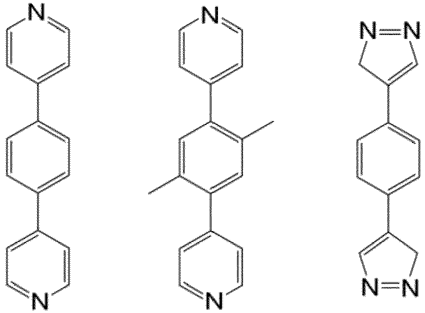

The organic ligand may be used alone or as a combination of two or more kinds thereof.

The content of the organic ligand in the metal-organic framework is preferably from 1 to 6, more preferably 1 to 3, and further preferably 1 or 2, with respect to the molar number of the metal ion.

The metal-organic framework may contain a component other than the metal ion and the crosslinkable organic ligand. Examples of the component include a crystal size modifier, a counter ion, a protein, a peptide, and DNA.

In the invention, in addition to the metal-organic framework described above, a covalent organic framework, an inorganic porous material, and an organic porous material may also be used as the porous material. The covalent organic framework is a crystalline or amorphous porous polymer framework having a network periodic structure formed by bonding light atoms, such as a hydrogen atom, a boron atom, a carbon atom, and an oxygen atom, via covalent bonds. The covalent organic framework also has a hard crystalline skeleton and pores having a nanosized small diameter, and thus has a prolonged spin-lattice relaxation time and can readily introduce the polarization source and the polarization object into the pores thereof and retain them in a dispersed state. The inorganic porous material used may be silica, organosilica, alumina, carbon, a metal oxide, and the like, and the organic porous material used may be a fiber aggregate containing entangled polymer fibers, and the like.

### (Condition of Pores of Porous Material)

The pore diameter of the pore of the porous material used in the invention is preferably from 0.2 to 1,000 nm, more preferably from 0.2 to 100 nm, and further preferably from 0.2 to 2 nm.

The pore diameter of the pore of the porous material may be measured by gas adsorption.

The porosity of the porous material is preferably from 1% to 90%, more preferably from 20% to 90%, and further preferably from 40% to 90%.

The porosity of the porous material may be measured by gas adsorption.

In the case where the pore diameter and the porosity of the porous material are in the ranges, the molecules of the polarization source and the polarization object can be readily introduced into the pores of the porous material in a sufficient amount, and can be retained with good dispersibility.

### [Additional Components]

The composition of the invention may be constituted only by the porous material and the polarization source, and may contain an additional component. For example, in the case where the molecule constituting the polarization source has an acidic group, a basic substance, such as sodium hydroxide and potassium hydroxide, is preferably added to the composition. In this manner, proton may be removed from the acidic group of the polarization source through ionization to form a conjugated base, which interacts with the metal ion of the metal-organic framework. According thereto, the amount of the polarization source introduced to the metal-organic framework can be significantly increased.

The composition of the invention may contain the polarization object, such as a protein, a peptide, and DNA, a solvent molecule, a template molecule, and the like, as the additional component. The polarization object herein is a substance capable of receiving the nuclear spin polarization formed in the polarization source and the porous material. For the description, the preferred range, and the specific examples of the polarization object, reference may be made to the corresponding description in the section <Polarization Enhancing Method>.

### <Introduction Amount of Polarization Source>

In the composition of the invention, the introduction amount of the polarization source to the porous material is preferably from 0.001% by mol to 1% by mol, more preferably from 0.001% by mol to 0.1% by mol, and further preferably from 0.01% by mol to 0.05% by mol, with respect to the molar number of a metal ion of the metal-organic framework.

The introduction amount of the polarization source to the porous material may be obtained in such a manner that the polarization source is taken out by decomposing the composition with hydrochloric acid or the like, and the peak intensity of the light absorption spectrum thereof is measured.

### [Spin-Lattice Relaxation Time T₁]

The spin-lattice relaxation time *T*₁ of the composition of the invention is preferably 1 second or more, more preferably 10 seconds or more, and further preferably 60 seconds or more. According thereto, the spin polarization of nuclei is further readily accumulated, and the polarization of the nuclear spins of the composition and the nuclear spins of the polarization object can be enhanced.

The spin-lattice relaxation time *T*₁ may be obtained by the saturation recovery method.

### <Composition for Dynamic Nuclear Polarization>

The composition for dynamic nuclear polarization of the invention will be described.

The composition for dynamic nuclear polarization of the invention contains the composition of the invention.

For the description, the preferred range, and the specific examples of the composition of the invention, reference may be made to the description in the section

### <Composition> above.

As described above, the composition of the invention has a long spin-lattice relaxation time and can readily introduce the polarization source and the polarization object, and thus can enhance the polarization of the nuclear spins thereof and the nuclear spins of the polarization object introduced. Therefore, the composition of the invention can be effectively used as a composition for dynamic nuclear polarization.

### <Highly Polarized Composition>

The highly polarized composition of the invention will be described.

The highly polarized composition of the invention is the composition of the invention that is highly polarized.

For the description, the preferred range, and the specific examples of the composition of the invention, and the method and the mechanism of the enhancement of the polarization thereof, reference may be made to the description in the section

### <Composition> above.

As described above, the composition of the invention has a long spin-lattice relaxation time, and thus can enhance the polarization of the nuclear spins of the entire thereof through dynamic nuclear polarization performed. According thereto, the highly polarized composition of the invention has nuclear spins having a high polarization over the entire thereof. Accordingly, by contacting the polarization object with the highly polarized composition, the spin polarization thereof is transferred to the polarization object, and the polarization of the nuclear spins of the polarization object can be enhanced.

The achievement of the "highly polarized composition" of the invention can be confirmed in such a manner that in the NMR spectrum thereof, a peak having a larger intensity (i.e., an enhanced peak) than the NMR spectrum of the composition in the thermal equilibrium state is observed. The enhanced peak intensity of the highly polarized composition is preferably 10 times or more, more preferably 100 times or more, and further preferably 1,000 times or more, the corresponding peak intensity of the composition in the thermal equilibrium state.

### <Polarization Enhancing Method>

The polarization enhancing method of the invention will be described.

The polarization enhancing method of the invention includes: contacting a substance with the highly polarized composition of the invention, or includes: contacting a substance with the composition of the invention; and then highly polarizing the composition to form a highly polarized composition.

In the description herein, the substance (i.e., the object of polarization), the nuclear spins of which are polarized by using the composition for dynamic nuclear polarization, and the substance (i.e., the object of polarization), the nuclear spins of which are polarized by contacting with the highly polarized composition of the invention or the composition of the invention, each are referred to as a "polarization object".

For the description of the composition, reference may be made to the section <Composition> above, and for the description of the highly polarized composition, reference may be made to the section <Highly Polarized Composition> above.

The polarization enhancing method of the invention may further include: transferring nuclear spin polarization of the highly polarized composition to the polarization object.

The nuclear spin polarization of the highly polarized composition can be transferred and diffused to the polarization object by contacting the polarization object with the highly polarized composition without any separate step performed, but at this time, a step for inducing the transfer of the nuclear spin polarization may be performed. Examples of the measure include a cross polarization method (CP method), a CP/MAS method using cross polarization, magic angle spinning, and broadband decoupling in combination, and adiabatic passage cross polarization, and the step is preferably performed by irradiation of a microwave.

The transfer of the nuclear spin polarization may be the transfer between the same nuclear species, such as the transfer from ¹H of the highly polarized composition to ¹H of the polarization object, may be the transfer between different nuclear species, such as the transfer from ¹H of the highly polarized composition to ¹⁹F of the polarization object, and may be both of them. The nuclear species of the polarization object receiving the nuclear spin polarization is not particularly limited, as far as the nuclear species has a spin quantum number I other than 0. Specific examples of the nuclear species include ¹H, ²H, ³He, ¹¹B, ¹³C, ¹⁴N, ¹⁵N, ¹⁷O, ¹⁹F, ²⁹Si, ³¹P, and ¹²⁹Xe, in which ¹H, ¹⁴N, ¹⁹F, and ³¹P are preferred since the natural abundances thereof are large, and ¹H and ¹⁹F are more preferred since the NMR signal intensities thereof are high.

The polarization object to be contacted with the composition for dynamic nuclear polarization is preferably a gas, a liquid, or a solution. By contacting the polarization object with the highly polarized composition of the invention, the polarization object readily enters into the pores of the porous material and encompassed therewith. Accordingly, the spin polarization of the nuclei of the highly polarized composition is transferred to the polarization object, and the polarization of the nuclear spin of the polarization object is enhanced.

The method of contacting the gas, the liquid, or the solution with the composition for dynamic nuclear polarization is not particularly limited, and for example, these materials may be injected to the composition, or these materials are filled in a vessel, and the vessel is disposed therein for impregnation.

In alternative, the polarization object in a state of dissolving or dispersing in a liquid may be mixed with the composition for dynamic nuclear polarization. At this time, composition for dynamic nuclear polarization may directly polarize the polarization object or may polarize via the liquid.

The polarization object preferably contains at least one kind of a compound selected from a hydrocarbon and a derivative of a hydrocarbon having at least one hydrogen atom substituted by a substituent.

The hydrocarbon may be an acyclic compound (aliphatic compound) or a cyclic compound, may be a saturated hydrocarbon or an unsaturated hydrocarbon, and may be a low molecular weight compound or a high molecular weight compound. The cyclic compound may be an alicyclic compound or an aromatic compound. The number of carbon atoms of the hydrocarbon is not particularly limited, and is generally in a range of from 1 to 10. The hydrocarbon may be a compound having a part of carbon atoms in the molecule substituted by a hetero atom. The hetero atom is not particularly limited, and examples thereof include N, P, O, and S.

In the derivative of a hydrocarbon, the substituent is not particularly limited, and at least one of the substituent is preferably a substituent containing an atom having a spin quantum number 1 other than 0, more preferably a substituent containing ¹³C, ¹⁵N, ¹⁹F, ²⁹Si, ³¹P, and the like, and further preferably a fluorine atom.

Specific examples of the polarization object are shown below. However, the polarization object capable of being used in the polarization enhancing method of the invention should not be construed as being limited to the specific examples.

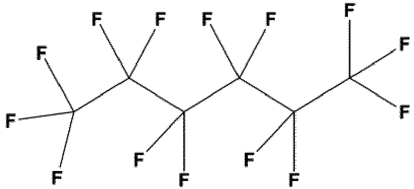

Preferred examples of the polarization object also include a biological component, such as a protein, a peptide, and DNA.

In the polarization enhancing method of the invention, the introduction amount of the polarization object to the highly polarized composition in the case where the porous material is the metal-organic framework is preferably 0.1% by mol to 500% by mol, more preferably from 1% by mol to 200% by mol, and further preferably from 10% by mol to 100% by mol, with respect to the molar number of the metal ion of the metal-organic framework.

The introduction amount of the polarization object to the highly polarized composition may be obtained by thermogravimetric analysis (TGA) and NMR measurement after digesting the metal-organic framework with an acid.

### <Highly Polarized Substance>

The highly polarized substance of the invention will be described.

The highly polarized substance of the invention is a substance that is highly polarized by the polarization enhancing method of the invention. For the description of the polarization enhancing method of the invention, reference may be made to the section <Polarization Enhancing Method> above. For the object substance to be highly polarized, reference may be made to the description, the preferred range, and the specific examples of the polarization object described in the section <Polarization Enhancing Method> above.

The highly polarized substance of the invention preferably has a polarization or 10⁻⁴ or more, more preferably 10⁻² or more, and further preferably 10⁻¹ or more.

The polarization of a substance can be measured by comparing the NMR signal intensity in the case where the enhancement of the polarization is performed and the signal intensity in the case where the enhancement is not performed.

### <NMR Measurement Method>

The NMR measurement method of the invention will be described. The NMR measurement method of the invention includes: measuring NMR (nuclear magnetic resonance) of a substance with the composition of the invention. The NMR measurement method is a concept that encompasses an MRI method.

For the description, the preferred range, and the specific examples of the composition of the invention, reference may be made to the section <Composition> above.

The NMR measurement method of the invention may be performed in such a manner that the measurement object of NMR is contacted with the highly polarized composition obtained by subjecting the composition of the invention to dynamic nuclear polarization, so as to enhance the polarization of nuclear spins of the measurement object, and then NMR of the measurement object is observed by a known NMR signal detecting method. In alternative, the method may be performed in such a manner that after introducing the measurement object of NMR to the composition of the invention, the polarization of the nuclear spins of the measurement object is enhanced by performing dynamic nuclear polarization, and then NMR of the measurement object is observed by a known NMR signal detecting method. In the case where the nuclear species to be observed for NMR is ¹³C or ¹⁹F, NMR is observed after transferring the polarization from the highly polarized ¹H to ¹³C or ¹⁹F through further irradiation with a microwave.

The steps of the method of introducing the measurement object of NMR to the composition of the invention and the enhancement of the polarization, reference may be made to the sections <Polarization Enhancing Method> and <Composition> above.

The NMR signal may be detected by a known method, such as the continuous-wave method and the pulse Fourier transform method, and for example, in the detection of the NMR signal by the pulse Fourier transform method, a device equipped with an RF coil (probe), an amplifier, and the like may be used.

In the invention, the polarization of the nuclear spins of the measurement object is enhanced with the composition of the invention, and thus the NMR signal from the measurement object can be detected with a high signal intensity. Therefore, the application of the NMR measurement method enables the analysis of structures and properties of compounds by the NMR spectroscopy and tests of biological organs by MRI performed with high sensitivity.

### Examples

The features of the invention will be described more specifically with reference to synthesis examples and examples below. The materials, the contents of treatments, the procedures of treatments, and the like shown below may be appropriately modified unless the modification deviates from the scope and sprit of the invention. Therefore, the scope of the invention is not construed as being limited to the specific examples shown below. The light absorption spectrum was measured with a spectrophotometer (V-670, produced by JASCO Corporation), the light emission spectrum was measured with a fluorescent spectrophotometer (LS 55, produced by PerkinElmer, Inc.), the transient decay curve of light emission was measured with a light emission lifetime measuring device (Quantaurus-Tau C11567-02, produced by Hamamatsu Photonics K.K.), and the spin-lattice relaxation time *T*₁ of proton (¹H) was obtained by the saturation recovery method. The dynamic nuclear polarization was performed in such a manner that the metal-organic framework having the polarization source introduced thereto was irradiated with excitation light of 589 nm and an electromagnetic wave of 18.1 GHz under application of an external magnetic field of 0.67 T.

### (Compounds used in Examples)

The polarization source and the ligands of the metal-organic framework used in the examples are shown below. In the following formulae, D represents ²H (deuterium).

### Polarization source

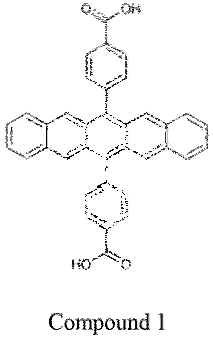

### Ligands of metal-organic framework

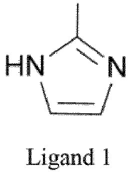

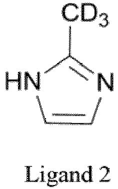

### Polarization object

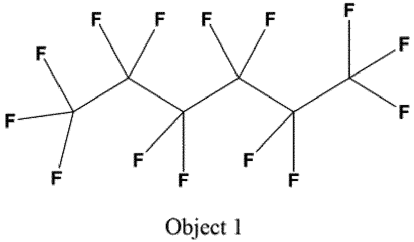

### [1] Synthesis of Compounds

The synthesis methods of the compound 1 and the ligand 2 used in the examples are shown below.

### (Synthesis Example 1)

### Synthesis of Compound 1

An intermediate 1 was synthesized by the following reaction.

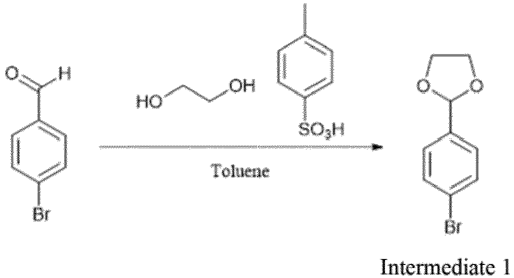

4-Bromobenzaidehyde (5.0 g, 27 mmol), ethylene glycol (3.3 g, 54 mmol), p-toluenesulfonic acid (0.18 g, 0.95 mmol), and toluene (75 mL) were placed in a vessel, and refluxed under heating for one day. The reaction liquid was neutralized with 50 mL of potassium carbonate aqueous solution, and then extracted three times with 50 mL of ethyl acetate. The collected organic layer was dried over magnesium sulfate and then filtered, and the solvent was evaporated under reduced pressure to provide a crude product in the form of an orange oil. The crude product was purified by column chromatography using a mixed solvent of dichloromethane and hexane = 1/1 as an eluent, so as to provide an intermediate 1 (2-(4-bromophenyl)-1,3-dioxotane) in the form of a yellow oil in a yield amount of 5.9 g and a yield of 95%.

An intermediate 2 was then synthesized by the following reaction.

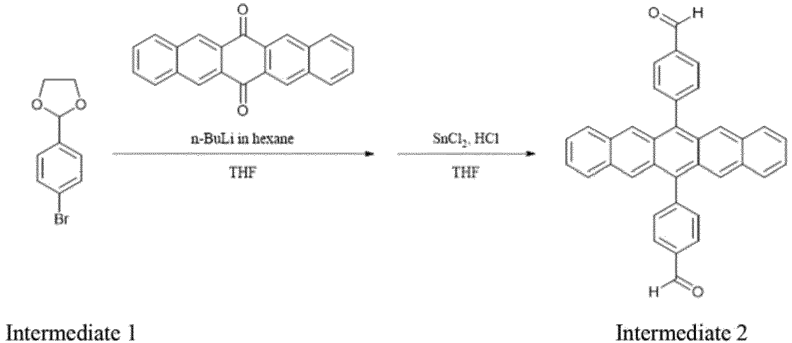

In a nitrogen atmosphere at -78°C, 5.74 mL of a 1.6 M hexane solution of n-butyl lithium (containing 9.2 mmol of n-butyl lithium) was added to 125 mL of a tetrahydrofuran solution containing the intermediate 1 (2.17 g, 9.5 mmol), followed by stirring for 60 minutes. 20 mL of a tetrahydrofuran solution containing 6,13-pentacenedione (973 mg, 3.2 mmol) was added to the mixture, followed by stirring at room temperature for 24 hours. 150 mL of a saturated aqueous solution of ammonium chloride was added to the reaction liquid to terminate the reaction, and then the reaction liquid was extracted with 100 mL of ethyl acetate three times. The collected organic layer was dried over magnesium sulfate and then filtered, and the solvent was evaporated under reduced pressure to provide a crude product in the form of a dark red oil. The crude product was diluted with 20 mL of tetrahydrofuran to provide a solution, to which tin(II) chloride dihydrate (2.01 g, 10.5 mmol) and concentrated hydrochloric acid (4 mL) were added, and after shielding light with an aluminum foil, the mixture was stirred at room temperature for 22 hours. 150 mL of water was added to the reaction liquid, which was extracted with 100 mL of chloroform three times. The collected organic layer was dried over magnesium sulfate and then filtered, and the solvent was evaporated under reduced pressure to provide a crude product. The crude product was purified by column chromatography using a mixed solvent of chloroform and hexane = 10/1 as an eluent, so as to provide an intermediate 2 (4,4'-(pentacen-6,13-diyl)dibenzaldehyde) in the form of dark violet powder in a yield amount of 0.48 g and a yield of 31%.

A compound 1 was then synthesized by the following reaction.

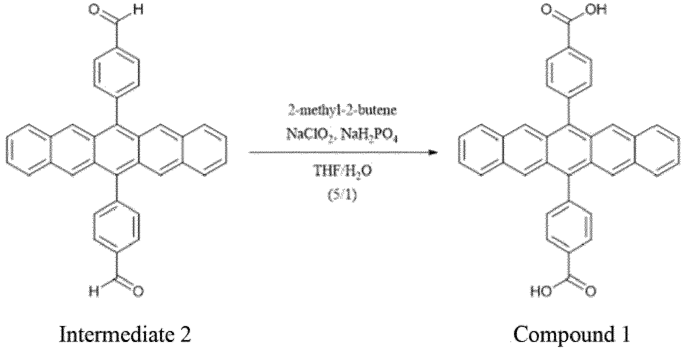

The intermediate 2 (1 g, 2.06 mmol), 2-methyl-2-butene (22 mL, 206 mmol), and 200 mL of tetrahydrofuran were placed in a flask, and stirred at 0°C for 5 hours or more to provide a suspension liquid. In an argon atmosphere, 40 mL of an aqueous solution containing sodium chlorite (745 mg, 8.24 mmol) and sodium dihydrogen phosphate (1,550 mg, 12.92 mmol) was added to the suspension liquid, and after shielding light with an aluminum foil, the mixture was gradually returned to room temperature under stirring for 12 hours. Tetrahydrofuran was evaporated from the reaction liquid under reduced pressure, and the water was added thereto, followed by filtering, so as to provide a crude product. The crude product was rinsed with chloroform and methanol to provide a compound 1 in the form of dark violet powder in a yield amount of 0.44 g and a yield of 41%.
¹H-NMR (400 MHz, DMSO-*d*₆, TMS) δ = 8.35 (d, 4H), 8.25 (s, 4H), 7.86 (q, 4H), 7.80 (d, 4H), 7.32 (q, 4H)
Elemental analysis: calculated for C₃₆H₂₂O₄ + H₂O = C₃₆H₂₄O₅, H: 4.51, C: 80.58; found H: 4.32, C: 80.43

### (Synthesis Example 2)

### Synthesis of Ligand 2

An intermediate 3 was synthesized by the following reaction.

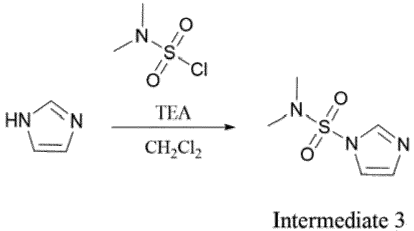

Tetraethylammonium (12.9 mL, 92.5 mmol) and *N*-dimethylsulfamoyl chloride (10 mL, 90.5 mmol) were added dropwise to a solution obtained by dissolving imidazole (5 g, 73.4 mmol) in anhydrous dichloromethane (50 mL). After stirring the mixture at room temperature for one day, a 10% potassium carbonate aqueous solution was added thereto, and the organic layer was separated. The organic layer was dried over sodium sulfate, and the solvent was evaporated in vacuum to provide a crude product in the form of an orange oil. The crude product was purified by column chromatography using a mixed solvent of chloromethane and methanol = 20/1 as an eluent, so as to provide an intermediate 3 (*N,N*-dimethylimidazole-1-sulfonamide) in the form of a yellow oil in a yield amount of 8.6 g and a yield of 67%.

An intermediate 4 was then synthesized by the following reaction.

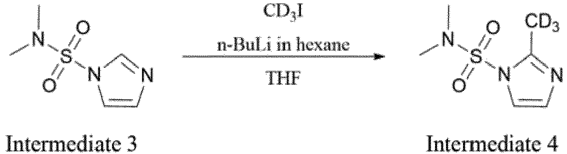

In a nitrogen atmosphere at -78°C, 36.3 mL of a 1.6 M hexane solution containing n-butyl lithium (58.1 mmol) was added dropwise to a solution obtained by dissolving the intermediate 3 (8.5 g, 48.5 mmol) in THF (300 mL), followed by stirring for 2 hours. Cooled iodomethane-*d*₃ (4.48 mL, 72.6 mmol) was added dropwise to the mixture, followed by stirring for one day. 150 mL of a 10% ammonium chloride aqueous solution was added to the reaction liquid to terminate the reaction, and then the reaction liquid was extracted with 50 mL of ethyl acetate three times. The solvent was evaporated from the collected organic layer to provide an intermediate 4 (*N,N*-dimethyl-2-(methyl-*d*₃)-imidazole-1-sulfonamide) in the form of an orange oil.

The ligand 2 was then synthesized by the following reaction.

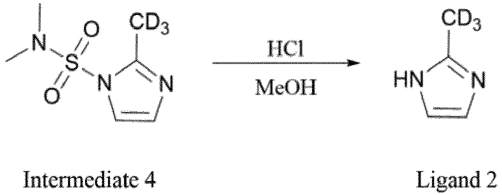

The intermediate 4 (8.6 g, 45.4 mmol) was refluxed in a 2 M hydrogen chloride methanol solution (280 mL) for 36 hours, and then the mixture was neutralized with a concentrated sodium hydrogen carbonate aqueous solution. The mixture was concentrated and treated with an ion exchange resin to provide a yellowish orange solid matter. The solid was recrystallized from toluene several times to provide the ligand 2 in the form of a colorless solid matter in a yield amount of 500 mg and a yield of 13%.
¹H-NMR (400 MHz, CDCl₃, TMS): δ = 6.95 (s, 2H)
Elemental analysis: calculated for C₄H₃D₃N₂ = H: 4.42, C: 41.08, N: 23.65; found H: 4.39, C: 40.90, N: 23.35

### [2] Production of Composition

### (Examples 1 to 6)

### Production of Composition using Polarization Source of Compound 1 and Metal-Organic Framework formed of Ligand 1 and Zinc Ion

6.5 mL of a methanol solution containing zinc nitrate hexahydrate Zn(NO₃)₂·6H₂O (225 mg, 0.76 mmol) was injected to 8.5 mL of a methanol solution containing the ligand 1 (250.5 mg, 3.05 mmol), and the compound 1 and sodium hydroxide in the amounts shown in Table 1, followed by stirring at room temperature for 1 hour. The resulting milky violet mixture was subjected to centrifugal separation to separate a crystalline solid matter, which was rinsed with methanol three times and then dried under reduced pressure to provide a composition 1 in the form of a crystalline solid matter in a yield amount of 56 mg and a yield of 32%.

### (Example 7)

### Production of Composition using Polarization Source of Compound 1 and Metal-Organic Framework formed of Ligand 2 and Zinc Ion

A composition was obtained in the same manner as in Example 6 except that the ligand 2 was used instead of the ligand 1.

### (Comparative Example 1)

### Production of Comparative Composition 1 as Metal-Organic Framework formed of Ligand 1 and Zinc Ion

A metal-organic framework was produced by performing the same procedures as in Example 6 except that the compound 1 and sodium hydroxide were not added to the methanol solution, and was designated as a comparative composition 1.

The introduction amounts of the compound 1 to the compositions produced in Examples are shown in Table 1. The light absorption spectra of the compositions having introduction amounts of the compound 1 thereto of 0.012% by mol, 0.027% by mol, and 0.13% by mol (i.e., the compositions of Examples 1 to 3) are shown in Fig. 2, the transient decay curves of fluorescent light emission of 620 nm with excitation light of 590 nm of the compositions are shown in Fig. 3, the ESR spectra measured by scanning the magnetic field under irradiation of excitation light of 589 nm and a microwave of the compositions are shown in Fig. 4, and the transient decay curves of the ESR signal observed at 680 mT of the composition are shown in Fig. 5. For comparison, the measurement results of a methanol solution of the compound 1 (concentration: 50 µM) and a solid of the compound 1 alone are shown in Fig. 2, and the measurement results of a chloroform solution of 6,13-diphenylpentacene (DPP) are shown in Figs. 4 and 5. The peak positions of NMR and the spin-lattice relaxation times *T*₁ measured after the dynamic nuclear polarization for the compositions produced in Examples 6 and 7 and Comparative Example 1 are shown in Table 2. The ¹H-NMR signal in the thermal equilibrium state observed at room temperature and the ¹H-NMR signal after the dynamic nuclear polarization for the composition of Example 7 are shown in Fig. 6. In Fig. 6, the signal intensity in the thermal equilibrium state is the accumulated intensity by accumulation 40 times, and the signal intensity after the dynamic nuclear polarization is the intensity by accumulation once.

**Table 1**

| Example No. | Charged amount | | Introduction amount of compound 1 (% by mol) |
|---|---|---|---|
| | Compound 1 (% by mol) | NaOH (% by mol) | |
| Example 1 | 0.4 | 0.8 | 0.012 |
| Example 2 | 0.8 | 1.6 | 0.027 |
| Example 3 | 4.0 | 8.0 | 0.130 |
| Example 4 | 4.0 | 0.0 | 0.026 |
| Example 5 | 4.0 | 4.0 | 0.470 |
| Example 6 | 0.25 | 10 | 0.036 |

The charged amounts and the introduction amounts in Table 1 each are shown as a molar percentage (% by mol) with respect to the molar number of the zinc ion Zn²⁺ forming the metal-organic framework.

**Table 2**

| Example No. | Peak position of NMR Spin-lattice relaxation spectrum time *T*₁ (ppm) (sec) | |
|---|---|---|
| Example 6 | 7.4 | 31 ± 0.1 |
| Comparative Example 1 | 7.4 | 25 ± 0.1 |
| Example 7 | 7.4 | 53 ± 1 |

The spin-lattice relaxation times *T*₁ in Table 2 each are shown as an average value ± a standard deviation in measurements three times.

As shown in Table 1, by adding sodium hydroxide and increasing the charged amount of the compound 1, the introduction amount of the compound 1 of 0.13% by mol was achieved. As shown in Table 2, there is not a large difference in spin-lattice relaxation time *T*₁ between the metal-organic framework produced with the addition of sodium hydroxide (i.e., the composition of Example 6) and the metal-organic framework produced without the addition of sodium hydroxide (i.e., the composition of Comparative Example 1), and it was found therefrom that the presence of sodium hydroxide added did not largely influence the spin-lattice relaxation time.

In the light absorption spectra in Fig. 2, the methanol solution of the compound 1 had an absorption peak at 593 nm, and the solid of the compound 1 alone had an absorption peak at 612 nm. The compositions of Examples 1 to 3 using the compound 1 and the metal-organic framework had absorption peaks in a range of from 603 to 607 nm. Herein, the absorption peak of the solid of the compound 1 alone can be understood as the absorption peak of the compound 1 in an aggregated state, and the absorption peak of the methanol solution of the compound 1 can be understood as the absorption peak of the compound 1 in a completely non-aggregated state (i.e., a completely dispersed state). In view of this, the fact that the compositions of the compound 1 and the metal-organic framework have absorption peaks in a range of from 603 to 607 nm means that the compound 1 exists in the metal-organic framework in a state where the compound 1 is dispersed to a certain extent. Furthermore, for the solid of the compound 1 alone, fluorescent light emission was not detected. This is estimated to mean that light extinction occurs due to the singlet splitting of the molecules in the solid of the compound 1 alone as an aggregate. On the other hand, as shown in Fig. 3, fluorescent light emission was observed for the compositions of Examples 1 to 3 using the compound 1 and the metal-organic framework. This also showed that the compound 1 existed in the metal-organic framework in a state where the compound 1 was dispersed to a certain extent.

In the ESR spectra in Fig. 4, ESR peaks were found at 685 mT. The ESR peak reflects the change of the energy state of the electron spins through resonance with the microwave, and from the transient decay curve spectra of the ESR signal shown in Fig. 5, it is found that the resonance lifetime thereof is in microsecond order. The lifetime of the excited triplet state is also in microsecond order, and agrees with the resonance lifetime observed herein. The resonance of the electron spins was derived from the compound 1 in the excited triplet state, and thus it was confirmed that the compound 1 was capable of functioning as a polarization source.

The compositions of Examples 6 and 7 using the compound 1 and the metal-organic framework showed strong enhancement of the ¹H-NMR signal detected after the dynamic nuclear polarization at the positions shown in Table 2. As shown in Fig. 6, the enhancement of the ¹H-NMR signal of the composition of Example 7 was particularly strong. On the other hand, the ¹H-NMR signal after the dynamic nuclear polarization of the composition of Comparative Example 1 was weaker than the compositions of Examples 6 and 7. The spin-lattice relaxation time *T*₁ was 31 seconds for the composition of Example 6 and 53 seconds for the composition of Example 7. That is, the far longer spin-lattice relaxation time *T*₁ was observed in the composition using the metal-organic framework having the organic ligand having a deuterated methyl group (Example 7) than the composition using the metal-organic framework having the organic ligand that was not deuterated (Example 6). It was found therefrom that the substitution of the substituent of the organic ligand by deuterium further prolonged the spin-lattice relaxation time *T*₁. The longer spin-lattice relaxation time *T*₁ means that the composition of Example 7 accumulates the spin polarization of the nuclei more readily than the composition of Example 6. It is estimated that the strong enhancement of the ¹H-NMR signal after the dynamic nuclear polarization is detected in the composition of Example 7 due to the effective accumulation of the spin polarization (see Fig. 6).

It was found from the above that by using the metal-organic framework as a substrate having a polarization source introduced thereto, the dynamic nuclear polarization system that readily introduced the polarization source and the polarization object thereto and had a long spin-lattice relaxation time was achieved.

### (Example 8)

### Introduction of Object 1 to Composition of Example 7 and Transfer of Nuclear Spin Polarization to Object 1

The composition produced in Example 7 (30 mg, 0.13 mmol) was immersed in a liquid of the object 1 (2 mL, 10 mmol) having been subjected to freeze deaeration, and immersed therein at room temperature for one day in a deaeration condition. Thereafter, the solid matter was recovered by drying under reduced pressure for 30 minutes, so as to provide a composition having the object 1 introduced thereto (i.e., an object-introduced composition) in the form of a crystalline solid matter.

The object-introduced composition produced in Example 8 had an introduction amount of the object 1 of approximately 2% by mol in terms of percentage by mol (% by mol) with respect to the molar number of the zinc ion Zn²⁺ forming the metal-organic framework.

Subsequently, the object-introduced composition was subjected to the dynamic nuclear polarization to provide a highly polarized composition. The object-introduced composition after the dynamic nuclear polarization was observed for the NMR spectrum, and the NMR signal of ¹H was confirmed at the same position and the similar intensity as in the case where the composition of Example 7 (i.e., the composition having no object 1 introduced) was subjected to the dynamic nuclear polarization.

Subsequently, for transferring the nuclear spin polarization from ¹H of the highly polarized metal-organic framework to ¹⁹F of the object 1, the object-introduced composition was irradiated with a microwave for cross polarization. The ¹⁹F-NMR signal of the object-introduced composition in the thermal equilibrium state measured at room temperature and the ¹⁹F-NMR signal thereof after the cross polarization (after transferring the nuclear spin polarization) are shown in Fig. 7. In Fig. 7, both the signal intensity in the thermal equilibrium state and the signal intensity after the dynamic nuclear polarization and the cross polarization are accumulated intensities after 100 times accumulation.

As shown in Fig. 7, the enhancement of the ¹⁹F-NMR signal was detected. ¹⁹F is a nuclear species that does not exist in the metal-organic framework formed of the ligand 2 and zinc ion and the compound 1 as the polarization source, and it is found therefrom that the ¹⁹F-NMR signal observed herein is derived from ¹⁹F of the object 1 introduced to the composition. It has been demonstrated by the above that the highly polarized composition of the invention can transfer the nuclear spin polarization to the polarization object encompassed therein, so as to achieve the enhancement of the polarization of the nuclear spins of the polarization object.

### Industrial Applicability

According to the invention, a dynamic nuclear polarization system that has a long spin-lattice relaxation time and can readily introduce the polarization source and the polarization object thereto can be provided. According thereto, the polarization of nuclear spins of various substances can be highly enhanced, and thereby the applicable range of NMR measurement can be enhanced, and simultaneously the analysis accuracy thereof can be improved through the enhancement of the sensitivity. Therefore, the invention has high industrial applicability.

## Claims

1. A composition comprising
(1) a porous material and
(2) a polarization source for dynamic nuclear polarization containing a molecule capable of being in an excited triplet state.

2. The composition according to claim 1, wherein the molecule capable of being in an excited triplet state is a compound having a skeleton containing from 4 to 6 benzene rings condensed to each other.

3. The composition according to claim 2, wherein the molecule capable of being in an excited triplet state is a compound having a pentacene skeleton.

4. The composition according to any one of claims 1 to 3, wherein the molecule capable of being in an excited triplet state is a compound constituted only by a carbon atom, a hydrogen atom, and a deuterium atom.

5. The composition according to any one of claims 1 to 4, wherein the porous material is a metal-organic framework or a covalent organic framework.

6. The composition according to claim 5, wherein the composition contains the metal-organic framework, the metal-organic framework has an organic ligand that has a ring structure substituted by a substituent, and at least one hydrogen atom of the substituent is substituted by deuterium.

7. The composition according to claim 5, wherein the composition contains the metal-organic framework, and the metal-organic framework has an organic ligand that has an imidazole skeleton.

8. The composition according to any one of claims 5 to 7, wherein the composition contains the metal-organic framework, and the metal-organic framework has a metal ion that contains a divalent to tetravalent metal ion.

9. The composition according to any one of claims 5 to 8, wherein the composition contains the metal-organic framework, and the metal-organic framework has a metal ion that contains a zinc ion Zn²⁺,

10. The composition according to any one of claims 5 to 9, wherein the composition contains the metal-organic framework, and the molecule capable of being in an excited triplet state has a functional group that interacts with a metal ion of the metal-organic framework.

11. The composition according to claim 10, wherein the functional group is an acidic group.

12. The composition according to claim 10, wherein the functional group is a carboxy group or a carboxylate anion group.

13. The composition according to any one of claims 1 to 12, wherein the molecule capable of being in an excited triplet state exists in pores of the porous material.

14. The composition according to any one of claims 5 to 13, wherein the composition has a content of the polarization source of 0.01% by mol or more with respect to the molar number of a metal ion of the metal-organic framework.

15. The composition according to any one of claims 1 to 14, wherein the composition has a spin-lattice relaxation time *T*₁ of 10 seconds or more.

16. The composition according to any one of claims 1 to 15, wherein the composition further comprises a substance capable of receiving nuclear spin polarization formed in the polarization source and the porous material.

17. A composition for dynamic nuclear polarization comprising the composition according to any one of claims 1 to 16.

18. A highly polarized composition comprising the composition according to any one of claims 1 to 16 that is highly polarized.

19. A polarization enhancing method of a substance, comprising: contacting the substance with the highly polarized composition according to claim 18, or comprising: contacting the substance with the composition according to any one of claims 1 to 16; and then highly polarizing the composition to form a highly polarized composition.

20. The polarization enhancing method according to claim 19, wherein the substance is a liquid or a solution.

21. The polarization enhancing method according to claim 19, wherein the substance is a gas.

22. The polarization enhancing method according to claim 20 or 21, wherein the substance is contacted with the highly polarized composition or the composition by impregnating the porous material constituting the highly polarized composition or the composition, with the substance.

23. The polarization enhancing method according to any one of claims 19 to 22, wherein the substance contains at least one kind of a compound selected from a hydrocarbon and a derivative of a hydrocarbon having at least one hydrogen atom substituted by a substituent.

24. The polarization enhancing method according to claim 23, wherein the substance contains the derivative of a hydrocarbon having at least one hydrogen atom substituted by a substituent, and at least one of the substituent contains an atom having a spin quantum number 1 other than 0.

25. The polarization enhancing method according to claim 23 or 24, wherein the substituent is a fluorine atom.

26. The polarization enhancing method according to any one of claims 19 to 25, wherein the method further comprises transferring nuclear spin polarization of the highly polarized composition to the substance.

27. The polarization enhancing method according to claim 26, wherein the nuclear spin polarization of the highly polarized composition is transferred to the substance by irradiating the highly polarized composition and the substance contacted with each other, with a microwave.

28. A highly polarized substance highly polarized by the method according to any one of claims 19 to 27.

29. An NMR measurement method comprising: measuring NMR of a substance with the composition according to any one of claims 1 to 16.
